# FASCICULE DE BREVET EUROPEEN

(11) **EP 0 973 022 B1**
(45) Date de publication et mention de la délivrance du brevet: **02.05.2003**
(21) Numéro de dépôt: 99401651.7
(22) Date de dépôt: 02.07.1999
(51) Int. Cl.: G01N 1/28, G01N 33/42

(54) **Presse à cisaillement giratoire**
Kreiselscherverdichter
Gyratory shear compacter

(30) Priorité: 15.07.1998 FR 9809036
(43) Date de publication de la demande: 19.01.2000
(73) Titulaire: LABORATOIRE CENTRAL DES PONTS ET CHAUSSEES, 75732 Paris Cedex 15 (FR)
(72) Inventeur: Vialletel, Hugues, 49100 Angers (FR); Gallier, Sylvain, 49100 Angers (FR); Gaschet, Jacky, 49250 Corne (FR); Moutier, Francis, 44830 Brains (FR)
(74) Mandataire: Le Bras, Hervé

(56) Documents cités:
- WO-A-98/21558
- GB-A- 911 155
- US-A- 5 275 056
- US-A- 5 323 655

## Description

L'invention concerne une presse à cisaillement giratoire utilisée pour compacter des échantillons de matériaux de compactage.

Elle concerne plus spécialement une presse destinée aux études de formulation des mélanges granulaires, à la mise au point de nouvelles formules et aux recherches concernant le processus de compactage de matériaux utilisés pour les revêtements routiers, afin de permettre la détermination du comportement au compactage des mélanges hydrocarbonés ainsi que celle des matériaux autres que ceux traités aux liants hydrocarbonés.

Dans ce type de presse, le compactage du matériau est obtenu par pétrissage sous une faible compression statique d'un échantillon de matériau contenu dans un moule cylindrique limité par des pastilles parallèles et maintenu à température constante, aux tolérances convenues de température près.

Le pétrissage par cisaillement est provoqué par le mouvement de l'axe du moule qui engendre une surface conique de révolution d'angle au sommet 2α pendant que les pastilles restent à tout moment perpendiculaires à l'axe de la surface conique.

La force résultante axiale F appliquée aux extrémités de l'échantillon, la température du moule et l'angle α sont maintenus constants pendant toute la durée de l'essai. La section et la masse de l'échantillon ne varient pas au cours de l'essai, mais la hauteur diminue continuellement.

Pour chaque échantillon, la hauteur et la force de cisaillement sont mesurées et mémorisées en fonction du nombre de girations.

US 5 275 056 et WO 95/22751 décrivent des presses à cisaillement giratoire dans lesquelles le moule comporte un anneau périphérique qui s'étend vers l'extérieur et qui est maintenu entre des galets disposés à des niveaux différents et qui sont entraînés en rotation autour d'un axe vertical et qui assurent le déhanchement du moule. Dans ces deux documents, les moyens de compression du matériau de l'échantillon sont disposés au-dessus du moule et ce dernier repose sur une table fixe. Ces dispositions entraînent des difficultés pour remplacer un moule contenant un échantillon par un deuxième moule contenant un autre échantillon, et pour réaliser des contrôles de plusieurs échantillons avec des paramètres différents, car le contrôle de l'angle α et son changement sont difficiles et longs.

US 5 323 655 concerne une presse à cisaillement giratoire dans laquelle les moyens de compression sont également disposés au-dessus du moule, ce dernier reposant sur une table tournante montée excentrée sur un plateau tournant. Ici il est nécessaire d'assurer un mouvement relatif entre la pastille inférieure du moule et la table tournante. Ceci est réalisé par un disque en céramique interposé entre la pastille inférieure et la table tournante.

Dans tous ces documents les moyens de compression de l'échantillon contenu dans le moule sont disposés dans la partie supérieure de l'appareil, ce qui augmente le volume et par le fait même la masse de l'appareil, et entraîne des difficultés pour positionner le moule avant le test et pour retirer l'échantillon après compactage.

Le but de la présente invention est de proposer une presse à cisaillement giratoire qui pallie ces inconvénients.

L'invention concerne donc une presse à cisaillement giratoire pour compacter des échantillons de matériaux de compactage, comportant
un châssis ;
un moule cylindrique d'axe X destiné à recevoir un échantillon interposé entre une pastille supérieure et une pastille inférieure maintenues parallèles entre elles ;
des moyens de compression de l'échantillon portés par ledit châssis et destinés à appliquer un effort constant sur l'une desdites pastilles selon un axe Y perpendiculaire auxdites pastilles ;
des moyens pour incliner l'axe X du moule par rapport à l'axe Y ;
des moyens de giration destinés à imprimer un mouvement giratoire de l'axe X autour de l'axe Y ;
des moyens de commande des moyens de compression et des moyens de giration ; et
des moyens de mesure des hauteurs de l'échantillon entre les pastilles et des efforts de cisaillement de l'échantillon en fonction du nombre de girations de l'axe X.

L'invention atteint son but par le fait que :
a) le châssis comporte dans sa partie supérieure un carter dont la rigidité est constante sur 360° et qui présente, dans sa paroi supérieure, une ouverture obturable par un couvercle destiné à servir d'appui pour la pastille supérieure, et, dans sa paroi inférieure, un orifice pour le passage d'un élément mobile des moyens de compression, qui est en appui sur la pastille inférieure ;
b) les moyens de giration comportent un manchon cylindrique externe d'axe Y monté rotatif dans le carter par l'intermédiaire de paliers, un manchon cylindrique interne d'axe X monté rotatif autour d'un contre-moule d'axe X destiné à recevoir le moule, par l'intermédiaire de paliers, et des moyens d'entraînement en rotation du manchon cylindrique externe portés par le carter ; le manchon cylindrique interne étant disposé à l'intérieur du manchon cylindrique externe et étant monté oscillant sur ce dernier de manière à pouvoir pivoter autour d'un axe Z perpendiculaire aux axes X et Y et situé sensiblement dans le plan de la pastille supérieure ;
c) les moyens pour incliner l'axe X du moule par rapport à l'axe Y comportent un système de réglage d'angle interposé en partie entre le manchon cylindrique externe et le manchon cylindrique interne et situé dans le plan médian perpendiculaire à l'axe Z.

Les avantageuses dispositions suivantes sont en outre adoptées :

La partie inférieure du châssis comporte une platine inférieure, une platine supérieure sur laquelle est fixé le carter, et une pluralité de colonnes parallèles à l'axe Y et reliant la platine inférieure à la platine supérieure, la platine inférieure supportant les moyens de compression.

Les moyens de compression comportent une vis sans fin entraînée par des deuxièmes moyens d'entraînement et un fourreau entraîné en translation selon l'axe Y par la vis sans fin et dont la tête est en appui sur la pastille inférieure, ledit fourreau étant guidé selon l'axe Y par des galets coopérant avec les colonnes et traversant une ouverture ménagée dans la platine supérieure avec interposition d'un coussinet. Une plaque à faible coefficient de friction est interposée entre la tête du fourreau et la pastille inférieure. Les deuxièmes moyens d'entraînement comportent un servomoteur électrique avec réducteur et un codeur de déplacement.

Le système de réglage d'angle est muni d'un vernier et une porte est ménagée dans la paroi périphérique du carter pour accéder au vernier. Les moyens de mesure des efforts de cisaillement comportent un capteur de cisaillement placé à l'intérieur du système de réglage d'angle. L'alimentation électrique du capteur de cisaillement et la transmission des données relevées par le capteur sont assurées par un collecteur tournant.

Le carter comporte un détecteur inductif pour le comptage du nombre de tours du manchon cylindrique externe.

Avantageusement la presse selon l'invention comporte en outre un système de manutention de moule, comprenant une potence disposée au-dessus de la paroi supérieure du carter et un treuil disposé au-dessus du couvercle et supporté par la potence.

D'autres avantages et caractéristiques de l'invention ressortiront à la lecture de la description suivante faite à titre d'exemple et en référence aux dessins annexés dans lesquels :

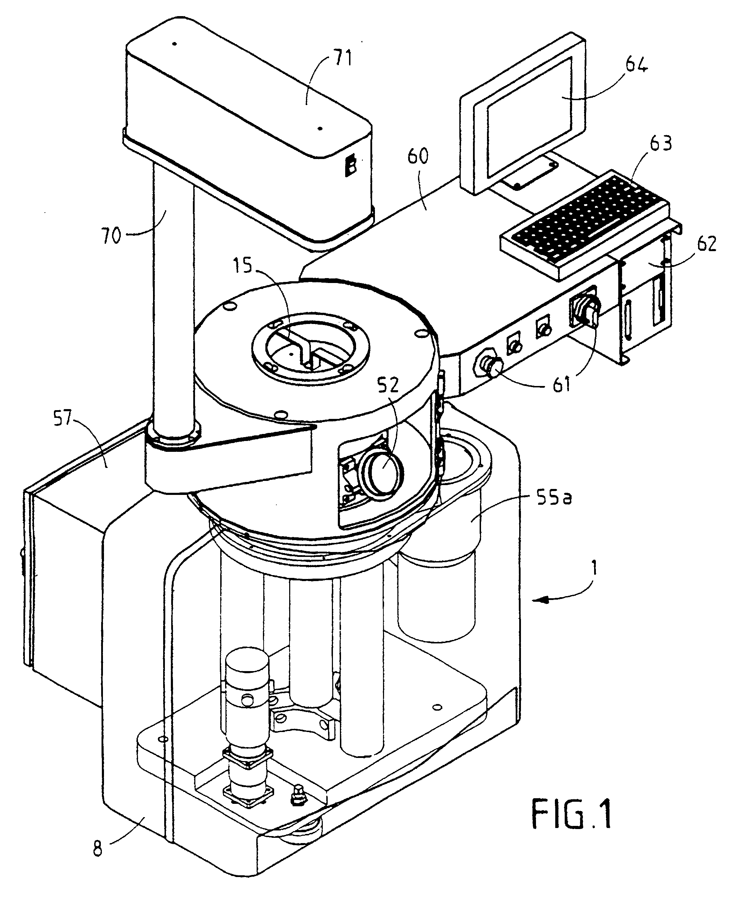

la figure 1 est une vue générale en perspective de la presse à cisaillement giratoire selon l'invention ;

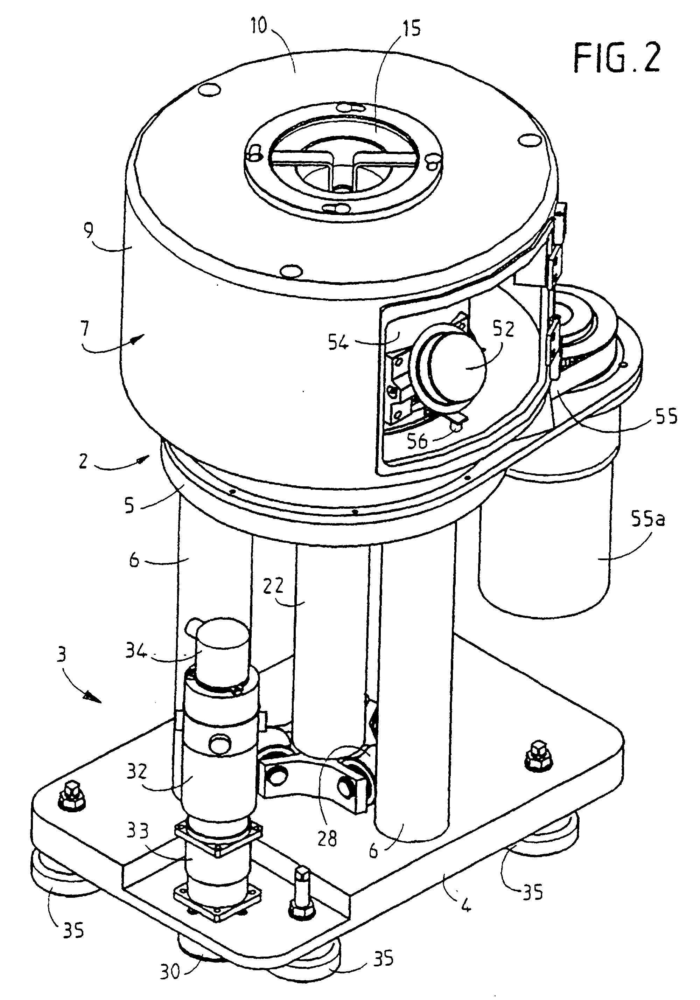

la figure 2 est une vue agrandie de la même machine dépouillée du capotage, de l'armoire électrique, du système de manutention du moule et de la table de commande ;

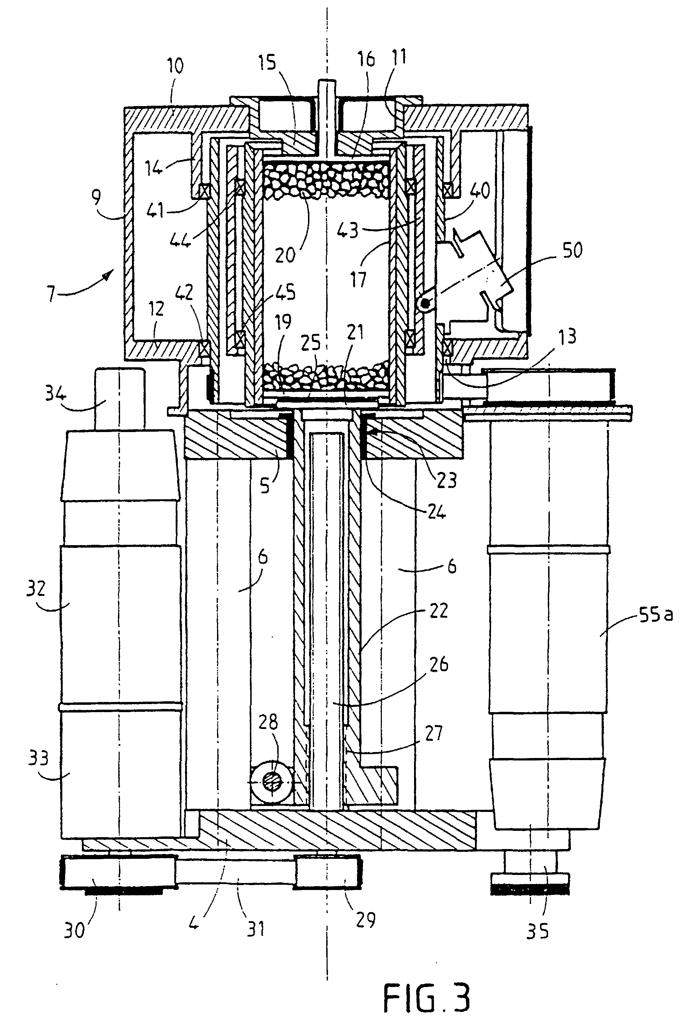

la figure 3 est une coupe de la machine selon un plan vertical médian ;

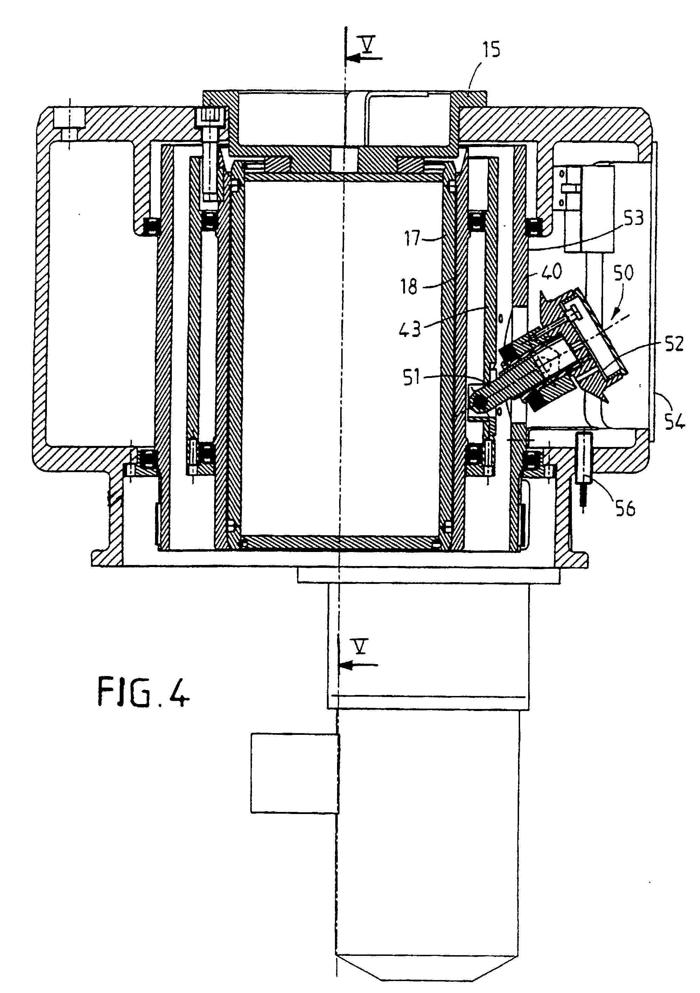

la figure 4 est une coupe de la partie supérieure de la machine selon un plan vertical médian représenté par la ligne IV-IV de la figure 5 ;
la figure 5 est une coupe de la partie supérieure de la machine selon un plan vertical représenté par la ligne V-V de la figure 4 ;

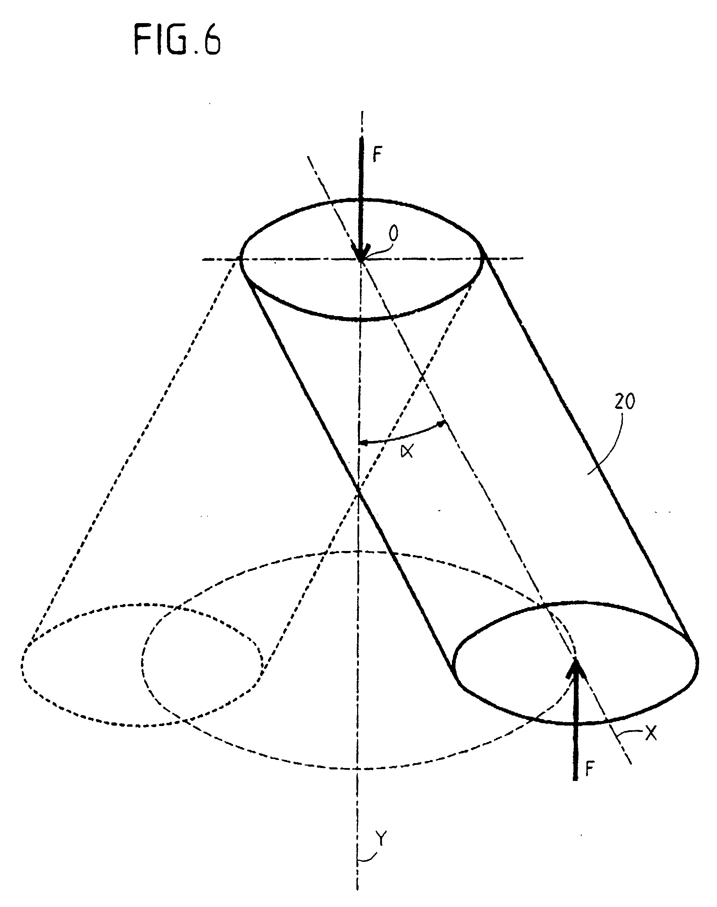

la figure 6 est une représentation schématique du mouvement de giration appliqué à l'échantillon de matériau.

Le dessin montre une presse à cisaillement giratoire 1 qui comporte un châssis 2 dont la partie inférieure 3 comprend une platine inférieure 4 et une platine supérieure 5 reliées par trois colonnes 6 et protégées par un capotage 8 et dont la partie supérieure 7 se présente sous la forme d'un carter rigide creux ayant une paroi périphérique 9 cylindrique de révolution autour d'un axe vertical Y, une paroi supérieure 10 comportant une ouverture 11 concentrique à l'axe Y et une paroi inférieure 12 fixée sur la platine supérieure 5 et présentant un passage 13 d'axe Y. La partie supérieure 7 comporte en outre un manchon 14 d'axe Y qui s'étend sous la paroi supérieure 10 en regard du passage 13. La rigidité du carter 7 est constante sur 360°.

L'ouverture 11 est susceptible d'être obturée par un couvercle 15 qui sert d'appui pour la face supérieure d'une pastille 16 disposée à l'extrémité supérieure d'un moule cylindrique 17 d'axe X monté coulissant dans un contre-moule cylindrique 18. A l'extrémité inférieure du moule 17 est prévue une pastille inférieure 19 qui peut coulisser dans le moule 17. L'espace du moule 17 délimité par la pastille supérieure 16 et la pastille inférieure 19 est rempli par un échantillon 20 d'un matériau de compactage, soumis d'une part à un effort de compression F exercé sur la pastille inférieure 19 selon la direction verticale Y, et à un mouvement giratoire de l'axe X autour de l'axe Y.

L'effort de compression F est exercé par la tête 21 d'un fourreau 22 d'axe Y qui traverse une ouverture 23 d'axe Y ménagée dans la platine supérieure 5, avec interposition d'un coussinet 24. Une plaque 25 d'un matériau à faible coefficient de friction est interposée entre la tête 21 et la face inférieure de la pastille inférieure 19. Le fourreau 22 est entraîné en translation verticale selon la direction de l'axe Y par une vis sans fin 26 montée sur la platine inférieure 4 et disposée à l'intérieur du fourreau 22, ce dernier présentant dans sa partie inférieure un filetage interne 27 coopérant avec le filetage externe de la vis sans fin. Le fourreau 22 est en outre guidé au cours de sa translation verticale par des galets 28 qui roulent sur la paroi externe des colonnes 6.

La vis sans fin 26 est entraînée en rotation, au moyen de poulies 29, 30 et d'une courroie 31, par un servomoteur 32 à couple constant entraînant un réducteur 33 sur lequel est montée la poulie 30.

Le servomoteur 32 est équipé d'un codeur d'angle 34, dont la mesure est représentative de la hauteur de l'échantillon 20 contenu dans le moule 17, entre la pastille inférieure 19 et la pastille supérieure 16. L'ensemble servomoteur 32 et réducteur 33 est disposé au-dessus de la platine inférieure 4, tandis que les poulies 29, 30 et la courroie 31 sont disposées sous la platine inférieure 4, cette dernière comportant une pluralité de pieds 35 réglables en hauteur.

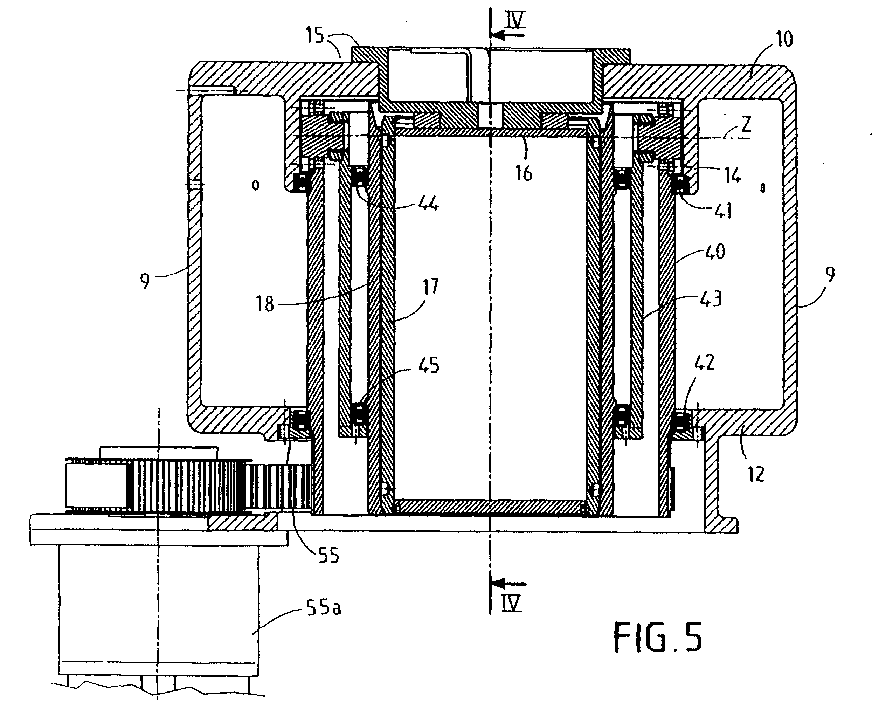

Le dispositif de mise en giration du moule 17 comporte un manchon cylindrique externe 40 d'axe Y monté rotatif à l'intérieur du carter 7 au moyen d'un premier palier 41 porté par le manchon 14 et d'un deuxième palier 42 monté à la périphérie du passage 13 de la paroi inférieure 12, et d'un manchon cylindrique interne 43 qui est disposé à l'intérieur du manchon externe 40 et monté sur ce dernier de manière à pouvoir osciller autour d'un axe horizontal Z situé sensiblement dans le plan de la face inférieure de la pastille supérieure 16, ainsi que cela est montré sur la figure 5.

Deux paliers 44 et 45 sont interposés entre le manchon cylindrique interne 43 et le contre-moule 18, afin de permettre la rotation de ces deux pièces l'une par rapport à l'autre, tout en empêchant leur déplacement relatif dans la direction de l'axe X du moule 17.

Un dispositif 50 de réglage de l'angle α entre l'axe X du moule 17 et l'axe vertical Y est prévu dans le plan vertical médian perpendiculaire à l'axe Z. Ce dispositif 50 est porté par le manchon externe 40 et coopère avec un système vis/écrou 51 solidaire du manchon interne. Il comporte un vernier 52 et un capteur d'effort dont l'alimentation électrique et la transmission de données sont assurées par un collecteur tournant 53.

Pour permettre l'accès au vernier 52 et le réglage de l'angle α, la paroi périphérique 9 du carter 7 est munie d'une porte d'accès 54.

Le manchon périphérique externe 40 est entraîné en rotation par un moteur électrique 55a entraînant une courroie crantée 55.

Le carter 7 est en outre équipé d'un détecteur inductif 56 servant au comptage du nombre de tours du manchon cylindrique externe 40.

Le vemier 52 permet un calage de l'angle α à 0°, ce qui permet de réaliser des éprouvettes à faces parallèles et perpendiculaires à l'axe X, de réaliser les contrôles de l'appareil, et un calage du même angle α de 30' à 2° par pas d'une minute dans un temps très bref, et sans moyens de contrôle.

Sur l'arrière de la partie inférieure 3 du bâti 2 est prévue une armoire électrique 57 qui assure l'alimentation électrique des moteurs d'entraînement 32 et 55a et des différents instruments de mesure.

Sur la droite du bâti 2 est fixée par vis une tablette 60 qui comprend les boutons 61 de mise en marche de la machine et qui supporte un micro-ordinateur 62 pilotant la machine. Au micro-ordinateur 62 est relié un clavier 63, qui permet l'introduction des caractéristiques des matériaux à contrôler, et un écran 64 pour l'affichage des résultats de chaque échantillon et éventuellement d'autres accessoires, tels que souris, dispositif de stockage, de données, etc... . Le micro-ordinateur 62 est relié au codeur d'angle 34 qui fournit la mesure de la hauteur de l'échantillon, au détecteur inductif 56 et au capteur d'effort placé à l'intérieur du dispositif de réglage de l'angle α, afin de permettre le stockage des données au cours de la giration du moule 17 et leur éventuel traitement informatique.

Sur la gauche du carter 7 peut être fixée une potence 70 qui supporte un treuil électrique 71 au-dessus du couvercle 15 pour la manutention du moule 17.

La presse à cisaillement giratoire décrite ci-dessus fonctionne de la manière suivante.

Un échantillon 20 de matériau est disposé dans le moule 17 entre les deux pastilles 16 et 19.

Le moule 17 rempli de matériau est mis dans le contre-moule 18 par l'ouverture supérieure 11, la tête 21 du fourreau 22 étant alors en position basse, c'est-à-dire légèrement au-dessus de la platine supérieure 5. Le couvercle 15 est ensuite fixé dans l'ouverture 11 par vissage. Au début de la série d'essais, le demi-angle α au sommet du cône de giration est affiché au moyen du vernier 52. Le servomoteur 32 à couple constant est mis en marche, ce qui entraîne un effort vertical croissant sur la pastille inférieure 19 qui met l'échantillon 20 en état de compression initiale. Lorsque la force de compression atteint la valeur F, le moteur 55a atteint sa vitesse d'essai nominale, et génère donc la rotation de l'axe X du moule autour de l'axe Y, l'axe X décrivant une surface conique dont le sommet 0 est situé au milieu de la face inférieure de la pastille supérieure 16. Cette giration provoque le pétrissage par cisaillement de l'échantillon 20. Pendant la giration du moule 17, la pastille supérieure 16 et la pastille inférieure 19 restent parallèles entre elles et perpendiculaires à l'axe vertical Y. Pendant tout la durée de l'essai, la force résultante axiale F appliquée aux extrémités de l'échantillon 20 et l'angle α sont maintenus constants dans des tolérances normalisées.

## Revendications

1. Presse à cisaillement giratoire pour compacter des échantillons (20) de matériaux de compactage, comportant
un châssis (2),
un moule cylindrique (17) d'axe X destiné à recevoir un échantillon (20) interposé entre une pastille supérieure (16) et une pastille inférieure (19) maintenues parallèles entre elles ;
des moyens de compression de l'échantillon portés par ledit châssis et destinés à appliquer un effort constant F sur l'une desdites pastilles selon un axe Y perpendiculaire auxdites pastilles ;
des moyens pour incliner l'axe X du moule par rapport à l'axe Y ;
des moyens de giration destinés à imprimer un mouvement giratoire de l'axe X autour de l'axe Y ;
des moyens de commande des moyens de compression et des moyens de giration ; et
des moyens de mesure des hauteurs de l'échantillon entre les pastilles et des efforts de cisaillement de l'échantillon en fonction du nombre de girations de l'axe X;
**caractérisée par le fait que** :
a) le châssis (2) comporte dans sa partie supérieure un carter (7) dont la rigidité est constante sur 360° et qui présente, dans sa paroi supérieure (10), une ouverture (11) obturable par un couvercle (15) destiné à servir d'appui pour la pastille supérieure (16), et, dans sa paroi inférieure (12), un orifice pour le passage d'un élément mobile (22) des moyens de compression, qui est en appui sur la pastille inférieure (19) ;
b) les moyens de giration comportent un manchon cylindrique externe (40) d'axe Y monté rotatif dans le carter (7) par l'intermédiaire de paliers (41, 42), un manchon cylindrique interne (43) d'axe X monté rotatif autour d'un contre-moule (18) d'axe X destiné à recevoir le moule (17), par l'intermédiaire de paliers (44, 45), et des moyens d'entraînement en rotation (55a) du manchon cylindrique externe (40) portés par le carter (7), le manchon cylindrique interne (43) étant disposé à l'intérieur du manchon cylindrique externe (40) et étant monté oscillant sur ce dernier de manière à pouvoir pivoter autour d'un axe Z perpendiculaire aux axes X et Y et situé sensiblement dans le plan de la pastille supérieure (19) ;
c) les moyens pour incliner l'axe X du moule (17) par rapport à l'axe Y comportent un système de réglage d'angle (50) interposé en partie entre le manchon cylindrique externe (40) et le manchon cylindrique interne (43) et situé dans le plan médian perpendiculaire à l'axe Z.

2. Presse selon la revendication 1, **caractérisée par le fait que** la partie inférieure (3) du châssis (2) comporte une platine inférieure (4), une platine supérieure (5) sur laquelle est fixé le carter (7), et une pluralité de colonnes (6) parallèles à l'axe Y et reliant la platine inférieure (4) à la platine supérieure (5), la platine inférieure (4) supportant les moyens de compression.

3. Presse selon la revendication 2, **caractérisée par le fait que** les moyens de compression comportent une vis sans fin (26) entraînée par des deuxièmes moyens d'entraînement (32) et un fourreau (22) entraîné en translation selon l'axe Y par la vis sans fin (26) et dont la tête (21) est en appui sur la pastille inférieure (19), ledit fourreau (22) étant guidé selon l'axe Y par des galets (28) coopérant avec les colonnes (6) et traversant une ouverture (23) ménagée dans la platine supérieure (5) avec interposition d'un coussinet (24).

4. Presse selon la revendication 3, **caractérisée par le fait qu'**une plaque (25) à faible coefficient de friction est interposée entre la tête (21) du fourreau (22) et la pastille inférieure (19).

5. Presse selon l'une quelconque des revendication 3 ou 4, **caractérisée par le fait que** les deuxièmes moyens d'entraînement comportent un servomoteur électrique (32) avec réducteur (33) et un codeur (34) de déplacement.

6. Presse selon l'une quelconque des revendications 1 à 5, **caractérisée par le fait que** le système de réglage d'angle (50) est muni d'un vernier (52), et **par le fait qu'**une porte (54) est ménagée dans la paroi périphérique (9) du carter (7) pour accéder au vernier (52).

7. Presse selon la revendication 6, **caractérisée par le fait que** les moyens de mesure des efforts de cisaillement comportent un capteur de cisaillement placé à l'intérieur du système de réglage d'angle (50).

8. Presse selon la revendication 7, **caractérisée par le fait que** l'alimentation électrique du capteur de cisaillement et la transmission des données relevées par le capteur sont assurées par un collecteur tournant (53).

9. Presse selon l'une quelconque des revendications 1 à 8, **caractérisée par le fait que** le carter (7) comporte un détecteur inductif (56) pour le comptage du nombre de tours du manchon cylindrique externe (40).

10. Presse selon l'une quelconque des revendications 1 à 9, **caractérisée par le fait qu'**elle comporte en outre un système de manutention de moule, comprenant une potence (70) disposée au-dessus de la paroi supérieure (10) du carter (7) et un treuil (71) disposé au-dessus du couvercle (15) et supporté par la potence (70).

## Claims

1. Gyratory shear press for compacting samples (20) of compaction materials, comprising
a chassis (2)
a cylindrical mould (17) of axis X adapted to receive a sample (20) interposed between an upper end (16) and a lower end (19), maintained parallel to each other;
means for compressing the sample, borne by said chassis and adapted to apply a constant effort (F) on one of said ends along an axis Y perpendicular to said ends;
means for inclining axis X of the mould with respect to axis Y;
gyration means adapted to impart a gyratory movement of axis X about axis Y;
means for controlling the compression means and the gyration means; and
means for measuring the heights of the sample between the ends and the shearing efforts of the sample as a function of the number of gyrations of axis X;
**characterised by** the fact that:
a) the chassis (2) comprises in its upper part a casing (7) whose rigidity is constant over 360° and which presents, in its upper wall (10), an opening (11) adapted to be closed by a lid (15) intended to serve as bearing for the upper end (16), and, in its lower wall (12), an orifice for the passage of a mobile element (22) of the compression means, which is in abutment on the lower end (19);
b) the gyration means comprise an outer cylindrical sleeve (40) of axis Y mounted to rotate in the casing (7) via bearings (41, 42), an inner cylindrical sleeve (43) of axis X mounted to rotate about a counter-mould (18) of axis X intended to receive the mould (17), via bearings (44, 45), and means for driving the outer cylindrical sleeve (40) in rotation (55a), said drive means being borne by the casing (7); the inner cylindrical sleeve (43) being disposed inside the outer cylindrical sleeve (40) and being mounted to oscillate thereon so as to be able to pivot about an axis Z perpendicular to axes X and Y and lying substantially in the plane of the upper end (19);
c) the means for inclining axis X of the mould (17) with respect to axis Y comprise an angle-adjusting system (50) partly interposed between the outer cylindrical sleeve (40) and the inner cylindrical sleeve (43) and lying in the median plane perpendicular to axis Z.

2. The press according to Claim 1, **characterised by** the fact that the lower part (3) of the chassis (2) comprises a lower plate (4), an upper plate (5) on which the casing (7) is fixed, and a plurality of columns (6) parallel to axis Y and connecting the lower plate (4) to the upper plate (5), the lower plate (4) supporting the compression means.

3. The press according to Claim 2, **characterised by** the fact that the compression means comprise an endless screw (26) driven by second drive means (32) and a sheath (22) driven in translation along axis Y by the endless screw (26), and whose head (21) is in abutment on the lower end (19), said sheath (22) being guided along axis Y by rollers (28) cooperating with the columns (6) and traversing an opening (23) made in the upper plate (5) with interposition of a bearing bush (24).

4. The press according to Claim 3, **characterised by** the fact that a plate (25) with a low coefficient of friction is interposed between the head (21) of the sheath (22) and the lower end (19).

5. The press according to Claim 3 or 4, **characterised by** the fact that the second drive means comprise an electric servomotor (32) with reduction gear (33) and a displacement encoder (34).

6. The press according to any one of Claims 1 to 5, **characterised by** the fact that the angle-adjusting system (50) is provided with a vernier (52) and by the fact that a door (54) is arranged in the peripheral wall (9) of the casing (7) in order to access the vernier (52).

7. The press according to Claim 6, **characterised by** the fact that the means for measuring the shearing efforts comprise a shear sensor placed inside the angle-adjusting system (50).

8. The press according to Claim 7, **characterised by** the fact that electrical supply of the shear sensor and transmission of the data picked up by the sensor are ensured by a rotating collector (53).

9. The press according to any one of Claims 1 to 8, **characterised by** the fact that the casing (7) comprises an inductive detector (56) for counting the number of revolutions of the outer cylindrical sleeve (40).

10. The press according to any one of Claims 1 to 9, **characterised by** the fact that it further presents a mould-handling system, comprising a bracket (70) disposed above the upper wall (10) of the casing (7) and a winch (71) disposed above the lid (15) and supported by the bracket (70).

## Patentansprüche

1. Kreiselscherverdichter zum Verdichten von Proben (20) aus Verdichtungsmaterial, mit
- einem Gestell (2);
- einer zylindrischen Form (17) mit einer Achse X, die dazu bestimmt ist, eine Probe (20) aufzunehmen, die zwischen einer oberen Platte (16) und einer unteren Platte (19) eingefügt ist, welche zueinander parallel gehalten werden;
- Mitteln zum Verdichten der Proben, welche von dem Gestell getragen werden und dazu bestimmt sind, einen ständigen Druck F auf eine der Platten entlang einer senkrecht zu den Platten verlaufenden Achse Y auszuüben;
- Mitteln zum Neigen der Achse X der Form in Bezug auf die Achse Y;
- Rotationsmitteln, die dazu bestimmt sind, die Achse X um die Achse Y in Drehung zu versetzen;
- Mitteln zum Steuern der Verdichtungsmittel und der Rotationsmittel; und
- Mitteln zum Messen der Probenhöhen zwischen den Platten und der Scherkräfte der Probe in Abhängigkeit von der Anzahl der Umdrehungen der Achse X;
**dadurch gekennzeichnet, dass**
a) das Gestell (2) in seinem oberen Teil ein Gehäuse (7) umfasst, dessen Widerstandsfähigkeit gegenüber Formveränderung auf 360° konstant ist und das in seiner oberen Wand (10) eine Öffnung (11), die durch einen Deckel (15) verschließbar ist, der dazu bestimmt ist, als Anlage für die obere Platte (16) zu dienen, und in seiner unteren Wand (12) eine Öffnung zum Durchtritt eines beweglichen Elements (22) der Verdichtungsmittel aufweist, das an der unteren Platte (19) anliegt;
b) die Rotationsmittel eine äußere zylindrische Muffe (40) mit eine Achse Y umfassen, die im Gehäuse (7) mit Hilfe von Lagern (41, 42) drehend montiert ist, sowie eine innere zylindrische Muffe (43) mit einer Achse X, die mit Hilfe von Lagern (44, 45) um eine zum Aufnehmen der Form (17) bestimmte Gegenform (18) mit einer Achse X drehend montiert ist, und Antriebsmitteln (55a), um die äußere zylindrische Muffe (40) in Drehung zu versetzen, welche vom Gehäuse (7) getragen werden, wobei die innere zylindrische Muffe (43) in der äußeren zylindrischen Muffe (40) angeordnet ist und an dieser schwenkbar in der Weise montiert ist, dass sie um eine Achse Z schwenken kann, die senkrecht zu den Achsen X und Y verläuft und im Wesentlichen in der Ebene der oberen Platte (19) angeordnet ist;
c) die Mittel zum Neigen der Achse X der Form (17) in Bezug auf die Achse Y ein System zur Winkeleinstellung (50) umfassen, das zum Teil zwischen die äußere zylindrische Muffe (40) und die innere zylindrische Muffe (43) eingefügt und in der Mittelebene senkrecht zur Achse Z angeordnet ist.

2. Verdichter nach Anspruch 1,
**dadurch gekennzeichnet, dass** der untere Teil (3) des Gestells (2) einen unteren Tisch (4), einen oberen Tisch (5), auf dem das Gehäuse (7) befestigt ist, und eine Vielzahl von Säulen (6) umfasst, die parallel zur Achse Y angeordnet sind und den unteren Tisch (4) mit dem oberen Tisch (5) verbinden, wobei der untere Tisch (4) die Verdichtungsmittel trägt.

3. Verdichter nach Anspruch 2,
**dadurch gekennzeichnet, dass** die Verdichtungsmittel eine endlose Schraube (26), die durch zweite Antriebsmittel (32) in Bewegung versetzt wird, und eine Hülse (22) umfassen, die durch die endlose Schraube (26) entlang der Achse Y in Bewegung versetzt wird und deren Kopf (21) an der unteren Platte (19) anliegt, wobei die Hülse (22) durch Rollen (28) entlang der Achse Y geführt wird, welche mit den Säulen (6) zusammenwirken, und eine Öffnung (23) durchquert, die in dem oberen Tisch (5) unter Einfügung einer Lagerbuchse (24) ausgebildet ist.

4. Verdichter nach Anspruch 3,
**dadurch gekennzeichnet, dass** zwischen dem Kopf (21) der Hülse (22) und der unteren Platte (19) eine Platte (25) mit geringem Reibungskoeffizienten eingefügt ist.

5. Verdichter nach einem der Ansprüche 3 oder 4,
**dadurch gekennzeichnet, dass** die zweiten Antriebsmittel einen elektrischen Servomotor (32) mit einer Untersetzung (33) und einen Wegcodierer (34) umfassen.

6. Verdichter nach einem der Ansprüche 1 bis 5,
**dadurch gekennzeichnet, dass** das System zur Winkeleinstellung (50) mit einem Nonius (52) versehen ist, und dass eine Türe (54) in der Umfangswand (9) des Gehäuses (7) angeordnet ist, um Zugang zu dem Nonius (52) zu gewähren.

7. Verdichter nach Anspruch 6,
**dadurch gekennzeichnet, dass** die Mittel zum Messen der Scherkräfte einen Schersensor umfassen, der innerhalb des Systems zur Winkeleinstellung (50) angeordnet ist.

8. Verdichter nach Anspruch 7,
**dadurch gekennzeichnet, dass** die Stromversorgung des Schersensors und die Übermittlung der von dem Sensor erfassten Daten durch einen drehenden Kollektor (53) erfolgen.

9. Verdichter nach einem der Ansprüche 1 bis 8,
**dadurch gekennzeichnet, dass** das Gehäuse (7) einen Induktionsdetektor (56) zum Zählen der Anzahl der Drehungen der äußeren zylindrischen Muffe (40) umfasst.

10. Verdichter nach einem der Ansprüche 1 bis 9,
**dadurch gekennzeichnet, dass** er zudem ein System zum Transportieren der Form umfasst, das einen über der oberen Wand (10) des Gehäuses (7) angeordneten Träger (70) und eine über dem Deckel (15) angeordnete und von dem Träger (70) getragene Winde (71) aufweist.
